Europäisches Patentamt

European Patent Office

Office européen des brevets

(19)

(11) Veröffentlichungsnummer: **0 009 146**
**B1**

(12) **EUROPÄISCHE PATENTSCHRIFT**

(45) Veröffentlichungstag der Patentschrift:
22.07.81

(21) Anmeldenummer: 79103194.1

(22) Anmeldetag: 29.08.79

(51) Int. Cl.³: **C 07 C 85/12**, C 07 C 87/10

(54) **Verfahren zur Herstellung von Di- und Triäthylamin.**

(30) Priorität: 08.09.78 DE 2839134

(43) Veröffentlichungstag der Anmeldung:
02.04.80 Patentblatt 80/7

(45) Bekanntmachung des Hinweises auf die Patenterteilung:
22.07.81 Patentblatt 81/29

(84) Benannte Vertragsstaaten:
DE FR GB IT NL

(56) Entgegenhaltungen:
DE-A-1 768 853
DE-A-2 519 838
US-A-3 264 354
US-A-3 673 251

(73) Patentinhaber: **EC ERDÖLCHEMIE GMBH,**
**Postfach 75 2002, D-5000 Köln 71 (DE)**

(72) Erfinder: **Mainusch, Klaus, Dr., Gneisenaustrasse 15a,**
**D-4047 Dormagen 1 (DE)**
Erfinder: **Schorsch, Reinhard,, Neckarstrasse 34,**
**D-4047 Dormagen 1 (DE)**
Erfinder: **Schleppinghoff, Bernhard, Dr.,**
**Adolf-Kolping-Strasse 5, D-4047 Dormagen (DE)**

(74) Vertreter: **Dill, Erwin, Dr. et al, c/o BAYER AG**
**Zentralbereich Patente Marken und Lizenzen Bayerwerk,**
**D-5090 Leverkusen 1 (DE)**

## Verfahren zur Herstellung von Di- und Triäthylamin

Die vorliegende Erfindung betrifft ein Verfahren zur kontinuierlichen Herstellung von Diäthylamin und Triäthylamin durch katalytische Hydrierung von Acetonitril in der Gasphase.

Das als Koppelprodukt bei der Acrylnitrilproduktion anfallende Acetonitril kann durch Hydrierung zu den Äthylaminen wirtschaftlich aufgewertet werden.

Gasphasenhydrierungen des Acetonitrils sind bereits bekannt. So wird in der US-PS 2 349 461 die Herstellung von insbesondere sekundären und/oder tertiären Aminen beschrieben. Danach wird Acetonitril zusammen mit 10 bis etwa 70 Gew.-% Acetaldehyd mit Wasserstoff an Nickel-Katalysatoren vorwiegend zu Di- und Triäthylamin umgesetzt. Nachteilig bei dem geschilderten Verfahren ist, daß das minderwertige Monoäthylamin in hohen Konzentrationen, z. B. bis zu 26 Gew.-%, entsteht. Ein weiterer entscheidender Nachteil ist die für das Verfahren erforderliche Anwesenheit von Acetaldehyd im Molverhältnis Nitril zu Aldehyd von bis zu 2 : 1.

In der GB-PS 1 239 505 wird die Hydrierung von Acetonitril bei einem Wasserstoffdruck von 36 bis 42 bar und einer Temperatur von 150°C (Beispiele 1 bis 3) an einem Chrom/Nickel-Katalysator beschrieben, wobei 2,3 bis 88% Monoäthylamin gebildet werden. Bei 5 bar/150°C und 230 bar/150°C (Beispiele 4 und 5) werden 68 bzw. 28% Monoäthylamin neben Di- und Triäthylamin erhalten. Nachteilig bei diesem Verfahren sind die geringe Raumgeschwindigkeit (Liquid hourly space velocity »LHSV«) von nur 0,33 Volumen Acetonitril pro Volumen Katalysator und Stunde »v/v.h« (600 ml/h Acetonitril pro 2 Liter Katalysator gemäß Beispiele 1—5) sowie der Zwangsanfall des minderwertigen Monoäthylamins. Auch erfordert die Anwendung der bei verhältnismäßig niedrigem Monoäthylamin-Abfall erforderlichen hohen Drücke erhöhte Investitionsausgaben, die sich als kostenmäßiger Nachteil auswirken.

Es wurde nun überraschend gefunden, daß die oben aufgeführten Nachteile bei der kontinuierlichen Herstellung von Diäthylamin und Triäthylamin durch katalytische Hydrierung von Acetonitril in der Gasphase bei Temperaturen von 115 bis 300°C und Drücken von 1 bis 60 bar vermieden werden können, wenn man die Hydrierung in Gegenwart eines Edelmetalls der VIII. Gruppe des Periodensystems (Mendelejew) auf Träger und in Gegenwart von etwa 2 bis 30 Gew.-% Monoäthylamin, bezogen auf Acetonitril, durchführt.

Die Trägerkatalysatoren können in jeder beliebigen Form (Kugeln, Zylinder, Extrudate, Hohlstränge, Granulate oder Schuppen) in einer Größe von 1 bis 8 mm, bevorzugt 3 bis 6 mm, Durchmesser eingesetzt werden. Die bevorzugte Form ist die Kugelform.

Als Träger sind alle üblichen Materialien, wie z. B. Aluminiumoxide, Kieselsäure, Aluminiumsilikate, Spinelle, Magnesiumoxid, Bariumsulfat oder Aktivkohle geeignet, die in stückiger Form eingesetzt werden können. Bevorzugt sind Aluminiumoxide mit einer spezifischen Oberfläche von 20 bis 400 m²/g oder die daraus hergestellten Spinelle, im besonderen Lithium-Aluminium-Spinell, für das erfindungsgemäße Verfahren geeignet.

Als Edelmetallkatalysatoren der VIII. Hauptgruppe seien genannt Rhodium, Palladium, Iridium oder Platin. Die genannten Metalle können allein oder als Gemisch miteinander eingesetzt werden. Man kann ihnen auch Vanadin und/oder Kupfer als Promotor zusetzen. Bevorzugt werden Palladium-, Platin- oder Palladium/Platin-Katalysatoren mit und ohne Vanadin-Zusätze verwendet. Besonders bevorzugt sind Palladium/Vanadin-, Platin/Vanadin- sowie Palladium/Platin/Vanadin-Katalysatoren für das erfindungsgemäße Verfahren geeignet. Im allgemeinen enthält der Trägerkatalysator 0,1 bis 5,0 Gew.-%, vorzugsweise 0,5 bis 2,0 Gew.-% des Edelmetalls der VIII. Gruppe des Periodensystems.

Falls zusätzlich Vanadin als Promotor-Komponente im Katalysator enthalten sein soll, so beträgt der Vanadin-Gehalt im fertigen Katalysator 0,1 bis 2,0 Gew.-%, bevorzugt 0,2 bis 0,8 Gew.-%.

Die für die Durchführung des erfindungsgemäßen Verfahrens verwendeten Katalysatoren sind an sich bekannt. Ihre Herstellung wird z. B. in der DE-OS 2 519 838 beschrieben.

Das Edelmetall, z. B. Palladium oder Platin, wird dabei auf den Träger in bekannter Weise aufgebracht. Beispielsweise wird das Edelmetall, z. B. Platin, in Mengen von 0,1 bis 5 Gew.-%, vorzugsweise 0,5 bis 2 Gew.-%, bezogen auf den fertigen Katalysator, auf den Träger aufgebracht. Zu diesem Zweck tränkt oder besprüht man den Träger beispielsweise mit einer wäßrigen Edelmetallsalz-, z. B. Platinsalzlösung. Für die Tränkung oder Besprühung sind alle handelsüblichen Edelmetallverbindungen geeignet. Vor der Reduktion der Edelmetallverbindungen in die metallische Form können die Edelmetallverbindungen zu Edelmetallhydroxid oder Edelmetalloxid umgewandelt werden. Die normalerweise nachfolgende Reduktion der Edelmetallverbindungen zum Metall kann beispielsweise mit Formaldehyd und Hydrazin in neutraler oder alkalischer Lösung oder mit Ameisensäure, Wasserstoff, Kohlenoxid oder Äthylen durchgeführt werden. Es können jedoch auch andere Reduktionsmethoden angewendet werden. Falls zusätzlich Vanadin als aktive Komponente im Katalysator enthalten sein soll, so kann das Vanadin vor oder nach dem Aufbringen der Hauptkomponente, z. B. des Edelmetalls, in Form von löslichen Vanadiumverbindungen, z. B. Vanadyloxalat, auf den Träger aufgebracht werden. Im allgemeinen wird sodann das auf den Träger aufgebrachte Vanadiumsalz durch Tempern bei Temperaturen von etwa 200 bis etwa 500°C in die Oxidform überführt. Der Gehalt an Vanadin im fertigen Katalysator kann 0,1 bis 2 Gew.-%, bevorzugt 0,2 bis 0,8 Gew.-%, betragen. Es ist zweckmäßig, vor Einsatz der so hergestellten Katalysatoren die von der Herstellung her enthaltenen Anionen mit destilliertem Wasser auszuwaschen.

Die Durchführung des erfindungsgemäßen Verfahrens kann im allgemeinen in der Weise erfolgen, daß man in einem Reaktor Wasserstoff und Acetonitril sowie das Monoäthylamin gasförmig durch den im Festbett angeordneten Katalysator leitet. Bei einem derartigen Hydrierreaktor kann es sich z. B. um einen Reaktor mit einem oder mehreren Reaktionsrohren handeln, die mit einem Kühlmittel umspült werden.

Für das erfindungsgemäße Verfahren eignet sich ein Druckbereich von 1 bis 60 bar, bevorzugt 1 bis 50 bar, besonders bevorzugt 5 bis 35 bar. Die Reaktionstemperatur beträgt erfindungsgemäß etwa 115 bis 300°C, bevorzugt etwa 130 bis 250°C, besonders bevorzugt etwa 135 bis 230°C.

Bei dem erfindungsgemäßen Verfahren werden im allgemeinen Mengen von etwa 460 bis 620 ml/h Acetonitril pro l Katalysator durchgesetzt. Das entspricht einer Raumgeschwindigkeit von etwa 0,5 bis 0,6 Volumen Acetonitril pro Volumen Katalysator und Stunde.

Als Einsatzprodukt in die Hydrierung eignet sich ein Acetonitril jeglicher Herkunft mit einer Reinheit von 90 bis 99,9 Gew.-%. Vorzugsweise wird Acetonitril eingesetzt, das als Nebenprodukt bei der Acrylnitril-Herstellung anfällt und eine Reinheit von etwa 95 bis 99,8 Gew.-% aufweist.

Die erfindungsgemäße Hydrierung des Acetonitrils wird in Gegenwart von 2 bis 30, bevorzugt 5 bis 25 Gew.-%, Monoäthylamin, bezogen auf Acetonitril, durchgeführt. Im allgemeinen verfährt man dabei so, daß man Acetonitril mit dem vorgenannten Reinheitsgrad in die Hydrierung einsetzt und das nach dem ersten Durchgang in einer Menge von etwa 2 bis 30, bevorzugt 5 bis 25 Gew.-% im Reaktionsgemisch enthaltene Monoäthylamin destillativ abtrennt und in die Hydrierung zurückführt. Diese Monoäthylamin-Abtrennung und -Rückführung erfolgt nach Anfahren der Hydrierung kontinuierlich.

Die Aufarbeitung kann kontinuierlich unter Anwendung an sich bekannter Methoden durchgeführt werden. Dazu kann in einer nachgeschalteten Kolonne nach Druckentspannung der im Reaktionsgemisch enthaltene Ammoniak abgestrippt werden oder in einer Druckdestillation als Kopfprodukt abgenommen und einer weiteren Verwendung zugeführt werden. Das flüssige Sumpfprodukt wird einer zweiten Destillationskolonne zugeführt, bei der Monoäthylamin als Destillat abgenommen und in den Hydrierreaktor zurückgeführt wird. Das flüssige Sumpfprodukt wird in einer dritten Destillationskolonne in reines Diäthylamin als Kopffraktion und in reines Triäthylamin getrennt, welches im Sumpf oder einige Böden darüber abgezogen werden kann.

Das erfindungsgemäße Verfahren gestattet selbstverständlich eine Regelung des Verhältnisses von Di- und Triäthylamin. Beispielsweise kann man bei einer Fahrweise mit Temperaturen von etwa 115 bis 170°C und Drücken von etwa 5 bis 20 bar vorwiegend Triäthylamin erhalten. Demgegenüber kann bei Durchführung des erfindungsgemäßen Verfahrens bei Temperaturen von etwa 170 bis 300°C und Drücken von etwa 20 bis 60 bar das Verhältnis von Diäthylamin zu Triäthylamin vergrößert werden.

Zusammenfassend läßt das erfindungsgemäße Verfahren folgende entscheidende Vorteile erkennen:

1.   Durch Verwendung der erfindungsgemäßen Katalysatoren kann die Hydrierung von Acetonitril bei überraschend niedrigen Drücken und sehr hohen Kontaktbelastungen durchgeführt werden.

2.   Eine ausschließlich und gezielte Gewinnung der wertvollen Produkte Diäthylamin und Triäthylamin wird durch eine Rückführung des anfallenden Monoäthylamins ermöglicht. Der Verbrauch einer Fremdchemikalie, z. B. Acetaldehyd, entfällt.

3.   Man kann durch Änderung lediglich der Reaktionstemperatur das Mengenverhältnis von Diäthylamin zu Triäthylamin zwischen 0,8 und 2,0 steuern.

Die nachfolgenden Beispiele sollen die erfindungsgemäße Arbeitsweise näher erläutern.

### A) Ohne Monoäthylamin-Rückführung (zum Vergleich)

#### Beispiele 1 — 6

Die Versuche wurden in der Gasphase in einem V2A-Rohrreaktor von 24 mm Durchmesser und 1,05 m Länge durchgeführt. Das Reaktionsrohr, das mit 242 g ( = 325 ml) eines Katalysators gefüllt war, wurde von einem Heiz- oder Kühlmedium durchströmt. Die verwendeten Katalysatoren bestanden aus Lithium-Aluminium-Spinell als Trägermaterial und wurden gemäß DE-OS 2 519 838 hergestellt. Die Metalldotierungen und spezifischen Oberflächen sind in Tabelle 1 angegeben. Durch das Reaktionsrohr wurden von oben her 170 ml/h 99%iges Acetonitril (LHSV = 0,52 v : v.h), welches ein aufgearbeitetes Nebenprodukt der Acrylnitril-Herstellung war, und ca. 220 Nl/h Wasserstoff gepumpt. Die mittlere Reaktionstemperatur betrug 145°C; der Druck betrug 16,9 ata. Das Reaktionsgemisch wurde bei Normaltemperatur kondensiert. Aus der Gasphase wurden 160 Nl/h Abgas entspannt, während 600 Nl/h als Kreisgas wieder in den Hydrierreaktor gelangten. Das vom Wasserstoff und gebildetem Ammoniak befreite Hydrierprodukt wurde analysiert. Die Ergebnisse sind in Tabelle 1 zusammengefaßt.

Tabelle 1

Erfindungsgemäße Katalysatoren und Äthylaminverteilung

| Beispiel | Katalysator aktives Metall in Gew.-% | Oberfl. (m²/g) | gebildetes Produkt in Gew.-% bezogen auf die Gesamtamine | | |
| --- | --- | --- | --- | --- | --- |
| | | | Mono- | Di- | Triäthylamin |
| 1 | 1,8% Pt | 120 | 5,8 | 42,7 | 51,5 |
| 2 | 1,8% Pd | 38 | 5,6 | 42,7 | 51,7 |
| 3 | 1,8% Pt/0,6% V | 340 | 6,2 | 42,4 | 51,4 |
| 4 | 1,8% Pd/0,6% V | 23 | 5,7 | 42,8 | 51,5 |
| 5 | 0,9% Pt/0,9% Pd | 290 | 5,3 | 43,0 | 51,7 |
| 6 | 0,9% Pt/0,9% Pd/0,6% V | 364 | 5,0 | 42,5 | 52,5 |

Der Umsatz an Acetonitril war vollständig. Das Verhältnis von Di- zu Triäthylamin im Hydrierprodukt betrug unter den angegebenen Hydrierbedingungen etwa 0,83. Die Versuche wurden nach jeweils 600 Betriebsstunden ohne jede Katalysatordesaktivierung abgebrochen.

Beispiel 7

Es wurde wie im Beispiel 4 verfahren, jedoch wurde der Versuch bei einer Reaktionstemperatur von 203° C durchgeführt. Aus dem Acetonitril wurde bei vollständigem Umsatz ein Äthylamingemisch mit folgenden Anteilen gebildet (Angaben in Gew.-%, bezogen auf die Gesamtamine):

| | |
| --- | --- |
| Monoäthylamin | 18,9% |
| Diäthylamin | 54,4% |
| Triäthylamin | 26,7% |

Das Verhältnis von Di- zu Triäthylamin im Hydrierprodukt betrug unter den angegebenen Reaktionsbedingungen 2,0. Auch nach 600 Betriebsstunden wurden dieselben Selektivitäten erreicht.

B) Mit Monoäthylamin-Rückführung (erfindungsgemäßes Verfahren)

Beispiel 8

Es wurde wie im Beispiel 4 verfahren, jedoch wurde der Hydrierreaktor mit 180 ml/h eines Gemisches aus 7,2 Gew.-% rückgeführtem Monoäthylamin und 92,8 Gew.-% Acetonitril beschickt. Bei vollständigem Acetonitril-Umsatz wurde ein Äthylamingemisch folgender Zusammensetzung gebildet (Anteile in Gew.-%, bezogen auf die Gesamtamine):

| | |
| --- | --- |
| Monoäthylamin | 3,1% |
| Diäthylamin | 45,4% |
| Triäthylamin | 51,5% |

Das Verhältnis von Di- zu Triäthylamin im Hydrierprodukt betrug hierbei 0,88. Das in den Reaktor zurückgeführte Monoäthylamin setzte sich bevorzugt zum Diäthylamin um. Nach 600 Betriebsstunden war keine Änderung der Äthylamin-Verteilung zu beobachten.

Beispiel 9

Es wurde wie im Beispiel 4 verfahren, jedoch wurde der Hydrierreaktor mit 180 ml/h eines Gemisches aus 16,1 Gew.-% rückgeführtem Monoäthylamin und 83,9 Gew.-% Acetonitril bei einer mittleren Reaktionstemperatur von 203° C beschickt. Bei vollständigem Acetonitril-Umsatz wurde ein Äthylamingemisch folgender Zusammensetzung gebildet (Anteile in Gew.-%, bezogen auf die Gesamtamine):

| | |
| --- | --- |
| Monoäthylamin | 15,2% |
| Diäthylamin | 52,9% |
| Triäthylamin | 31,9% |

Das Verhältnis von Di- zu Triäthylamin im Hydrierprodukt betrug hierbei 1,66. Das in den Reaktor rückgeführte Monoäthylamin setzte sich bevorzugt zum Diäthylamin um. Nach 600 Betriebsstunden wurde der Versuch ohne Katalysatordesaktivierung abgebrochen.

**Patentansprüche**

1. Verfahren zur kontinuierlichen Herstellung von Diäthylamin und Triäthylamin durch katalytische Hydrierung von Acetonitril in der Gasphase bei Temperaturen von 115 bis 300° C und Drücken von 1 bis 60 bar, dadurch gekennzeichnet, daß man die Hydrierung in Gegenwart eines Edelmetalls der VIII. Gruppe des Periodensystems (Mendelejew) auf einem Träger und in Gegenwart von etwa 2 bis 30 Gew.-% Monoäthylamin, bezogen auf Acetonitril, durchführt.

2. Verfahren nach Anspruch 1, dadurch gekennzeichnet, daß man das im Reaktionsgemisch enthaltene Monoäthylamin destillativ abtrennt und in die Hydrierung zurückführt.

3. Verfahren nach Ansprüchen 1 und 2, dadurch gekennzeichnet, daß man Acetonitril in die Hydrierung einsetzt und das nach dem ersten Durchgang in einer Menge von etwa 2 bis 30 Gew.-% im Reaktionsgemisch enthaltene Monoäthylamin destillativ abtrennt und in die Hydrierung zurückführt, wobei sodann die Monoäthylamin-Abtrennung und -Rückführung kontinuierlich vorgenommen wird.

4. Verfahren nach Ansprüchen 1 bis 3, dadurch gekennzeichnet, daß man die Hydrierung an einem Trägerkatalysator durchführt, der 0,1 bis 5 Gew.-% eines Edelmetalls der VIII. Gruppe des Periodensystems enthält.

5. Verfahren nach Ansprüchen 1 bis 4, dadurch gekennzeichnet, daß man die Hydrierung in Gegenwart eines zusätzlichen Vanadin enthaltenden Katalysators durchführt.

6. Verfahren nach Ansprüchen 1 bis 5, dadurch gekennzeichnet, daß man die Hydrierung in Gegenwart eines Katalysators durchführt, der zusätzlich 0,1 bis 2,0 Gew.-% Vanadin enthält.

7. Verfahren nach Ansprüchen 1 bis 6, dadurch gekennzeichnet, daß man die Hydrierung in Gegenwart eines 0,5 bis 2,0 Gew.-% Palladium und/oder Platin und gegebenenfalls 0,2 bis 0,8 Gew.-% Vanadin enthaltenden Katalysators durchführt.

8. Verfahren nach Ansprüchen 1 bis 7, dadurch gekennzeichnet, daß man die Hydrierung in Gegenwart eines Trägerkatalysators durchführt, bei dem der Träger ein Lithium-Aluminiumspinell mit einer spezifischen Oberfläche von 20 bis 400 m$^2$/g ist.

9. Verfahren nach Ansprüchen 1 bis 8, dadurch gekennzeichnet, daß man in die Hydrierung Acetonitril einsetzt, das als Nebenprodukt bei der Acrylnitril-Herstellung anfällt.

## Claims

1. Process for the continuous preparation of diethylamine and triethylamine by catalytic hydrogenation of acetonitrile in the gas phase at temperatures of 115°C to 300°C and under pressures of 1 to 60 bars, characterised in that the hydrogenation is carried out in the presence of a noble metal of group VIII of the periodic table (Mendeleeff) on a support and in the presence of about 2 to 30% by weight of monoethylamine, based on acetonitrile.

2. Process according to Claim 1, characterised in that the monoethylamine contained in the reaction mixture is separated off by distillation and recycled into the hydrogenation.

3. Process according to Claims 1 and 2, characterised in that acetonitrile is employed for the hydrogenation and the monoethylamine which after the first pass is present in the reaction mixture in an amount of about 2 to 30% by weight is separated off by distillation and recycled into the hydrogenation, the separating off and recycling of monoethylamine then being carried out continuously.

4. Process according to Claims 1 to 3, characterised in that the hydrogenation is carried out on a supported catalyst which contains 0.1 to 5% by weight of a noble metal of group VIII of the periodic table.

5. Process according to Claims 1 to 4, characterised in that the hydrogenation is carried out in the presence of a catalyst which additionally contains vanadium.

6. Process according to Claims 1 to 5, characterised in that the hydrogenation is carried out in the presence of a catalyst which additionally contains 0.1 to 2.0% by weight of vanadium.

7. Process according to Claims 1 to 6, characterised in that the hydrogenation is carried out in the presence of a catalyst which contains 0.5 to 2.0% by weight of palladium and/or platinum and optionally 0.2 to 0.8% by weight of vanadium.

8. Process according to Claims 1 to 7, characterised in that the hydrogenation is carried out in the presence of a supported catalyst in which the support is a lithium aluminium spinel with a specific surface area of 20 to 400 m$^2$/g.

9. Process according to Claims 1 to 8, characterised in that acetonitrile which is obtained as the by-product from the production of acrylonitrile is employed in the hydrogenation.

## Revendications

1. Procédé de production continue de diéthylamine et de triéthylamine par hydrogénation catalytique d'acétonitrile en phase gazeuse à des températures de 115 à 300°C et à des pressions de 1 à 60 bars (0,1 à 6,0 MPa), caractérisé en ce qu'on conduit l'hydrogénation en présence d'un métal noble du groupe VIII du Système Périodique (Mendeléeff) sur un support et en présence d'environ 2 à 30% en poids de mono-éthylamine, par rapport à l'acétonitrile.

2. Procédé suivant la revendication 1, caractérisé en ce qu'on sépare par distillation la mono-éthylamine contenue dans le mélange réactionnel et qu'on la recycle dans l'hydrogénation.

3. Procédé suivant les revendications 1 et 2, caractérisé en ce qu'on utilise de l'acétonitrile dans l'hydrogénation et on sépare par distillation la mono-éthylamine contenue dans le mélange réactionnel en quantité d'environ 2 à 30% en poids après le premier passage et on la recycle dans l'hydrogénation, la séparation et le recyclage de la mono-éthylamine étant ensuite effectués en continu.

4. Procédé suivant les revendications 1 à 3, caractérisé en ce qu'on conduit l'hydrogénation sur un catalyseur fixé sur un support qui renferme 0,1 à 5% en poids d'un métal noble du groupe VIII du Système Périodique.

5. Procédé suivant les revendications 1 à 4, caractérisé en ce qu'on conduit l'hydrogénation en présence d'un catalyseur additionnel contenant du vanadium.

6. Procédé suivant les revendications 1 à 5, caractérisé en ce qu'on conduit l'hydrogénation en présence d'un catalyseur qui contient en outre 0,1 à 2,0% en poids de vanadium.

7. Procédé suivant les revendications 1 à 6, caractérisé en ce qu'on conduit l'hydrogénation en présence d'un catalyseur contenant 0,5 à 2,0% en poids de palladium et/ou de platine et, le cas échéant, 0,2 à 0,8% en poids de vanadium.

8. Procédé suivant les revendications 1 à 7, caractérisé en ce qu'on conduit l'hydrogénation en présence d'un catalyseur fixé sur un support dont le support est un spinelle de lithium et d'aluminium ayant une surface spécifique de 20 à 400 m²/g.

9. Procédé suivant les revendications 1 à 8, caractérisé en ce qu'on utilise dans l'hydrogénation de l'acétonitrile qui est obtenu comme sous-produit dans la production de l'acrylonitrile.